(19) Europäisches Patentamt
European Patent Office
Office européen des brevets

(11) Publication number: **0 399 986 A1**

# (12) EUROPEAN PATENT APPLICATION

(21) Application number: 90870077.6

(22) Date of filing: 21.05.90

(51) Int. Cl.[5] C07D 295/20, C07C 333/16, C08L 21/00, C08K 5/39

(30) Priority: 22.05.89 US 354953

(43) Date of publication of application: 28.11.90 Bulletin 90/48

(84) Designated Contracting States: AT BE CH DE DK ES FR GB GR IT LI LU NL SE

(71) Applicant: MONSANTO COMPANY
800 North Lindbergh Boulevard
St. Louis Missouri 63167(US)

(72) Inventor: Faulkner, Roger Webster
1 Costley Court
Kent, Ohio 44240(US)
Inventor: Rostek, Charles John, Jr.
413 Miles Road
Bentleyville, Ohio 44022(US)

(74) Representative: Ernst, Hubert et al
Monsanto Services International S.A., Patent Department, Avenue de Tervuren 270-272, Letter Box No. 21
B-1150 Brussels(BE)

## (54) Zwitterionic tertiary ammonium dithiocarbamates.

(57) Zwitterionic tertiary ammonium dithiocarbamates are provided by the reaction of carbon disulfide with a polyamine and can be utilized as either a primary or a secondary accelerator in a sulfur vulcanizable rubber composition. The polyamine contains at least one secondary and at least one tertiary amine with the reaction product containing a tertiary ammonium cation and the carbon disulfide becomes part of a N,N-dialiphatic dithiocarbamate anion which is covalently connected to said tertiary ammonium cation. Such compounds are readily prepared via a direct route by the reaction of the polyamine with carbon disulfide in the presence of a non-hydroxylic solvent or in a gas. The reaction product solid can be readily removed from the solvent as by filtration. When utilized as accelerators, the compounds of the present invention yield improved vulcanization parameters.

EP 0 399 986 A1

# ZWITTERIONIC TERTIARY AMMONIUM DITHIOCARBAMATES

## FIELD OF THE INVENTION

The present invention relates to zwitterionic tertiary ammonium dithiocarbamates as sulfur vulcanization accelerators for rubber, and/or synthetic intermediates in the synthesis of conventional dithiocarbamate derivatives. The zwitterionic compounds are made by the reaction of carbon disulfide ($CS_2$) with a polyamine containing at least one secondary and at least one tertiary amine wherein the reaction product contains a tertiary ammonium cation and the $CS_2$ becomes part of a N,N-dialiphatic dithiocarbamate anion which is covalently connected to the aforesaid ammonium cation.

## BACKGROUND

Heretofore, numerous compounds have existed which were utilized as accelerators for sulfur vulcanized rubbers. In particular, N,N-dialiphatic dithiocarbamate salts such as metal or ammonium salts, certain esters, the related thiuram monosulfides and disulfides, and dithiocarbamate sulfenamides derived from simple secondary amines, have long been known as so-called "ultra accelerators" in the sulfur vulcanization of rubber.

Dithiocarbamate derivatives which also contain one or more tertiary amine groups covalently attached to the dithiocarbamate group are also known in the prior art. For example, U.S. Patent No. 4.687,756 relates to vulcanization accelerators having at least one dithiocarbamoyl group which is covalently attached to a tertiary amine group in the same molecule or ion. U. S. Patent No. 4,687,756 relates to the same general types of N,N-disubstituted dithiocarbamate derivatives as were listed in the above paragraph, in the sense that the dithiocarbamate groups thereof are either nonionized (as in the thiuram sulfides), or are associated with a metallic counterion.

British Patent No. 1,428,527 relates to N,N-dialkyldithiocarbamate esters which contain three tertiary amine groups covalently attached thereto. As in the case of the above cited U.S. Patent 4,687,756, the tertiary amine groups of these dithiocarbamic acid aminomethyl esters are not normally ionized (i.e., protonated). The compounds of U.S. Patent No. 4,687,756 are more active than those of British Patent No. 1,428,527 derived from the same amine because only one of the two secondary amine groups used to make the compounds of British Patent No. 1,428,527 can be released from the molecule as a dithiocarbamate, whereas all the secondary amine groups used to make the compounds of U.S. Patent No. 4,687,756 can be released from those compounds during conventional sulfur vulcanization of unsaturated elastomers as dithiocarbamates, because of the stoichiometry of these various molecules. This is also usually a feature of the zwitterionic dithiocarbamates of the present invention; that is, the stoichiometry between $CS_2$ and basic secondary amine groups is one to one in the present invention so that each of the secondary amine groups used in the synthesis can, in most cases, enter into the acceleration system as a dithiocarbamate.

The compounds of this invention differ from the prior art in that the dithiocarbamate anion and its tertiary ammonium counterion are present in the same molecule. The empirical formulas of the compounds of this invention are very close to those of thiuram disulfides derived from the same amine, but differ at least by having one more hydrogen atom. The actual molecular weights of the compounds of this invention differ substantially from the related prior art compounds derived from the same amines, including the corresponding thiuram disulfides.

The distinctions between U.S. Patent No. 4,687,756, British Patent 1,428,527, and the present invention can be clearly illustrated by considering examples of the specific compounds which are derived by means of the various inventions from the same amine. 1-methyl-piperazine has been chosen to illustrate the various inventions because it is a preferred amine for all three inventions. (1-methylpiperazine leads to high activity dithiocarbamate accelerators which damage polyester cord much less during vulcanization than do conventional dithiocarbamates, see U.S. Patent No. 4,687,756). Also, the N-nitrosamine derivative of this amine is reportedly much less carcinogenic in animal assays than are the nitrosamines from the amines used in the commercially significant dithiocarbamate accelerators of today (see for example German Patent Application No. P3029 318.6).

| Compound No. | Empirical Formula | Chemical Formula |
|---|---|---|
| (1) | $C_6H_{11}N_2S_2$ | $C_{12}H_{22}N_4S_4$ |

Structure and General Name

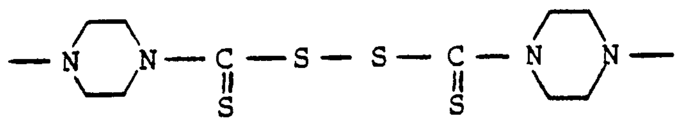

thiuram disulfide

| (2) | $C_{12}H_{22}N_2S_3$ | $C_{12}H_{22}N_4S_4$ |
|---|---|---|

Structure and General Name

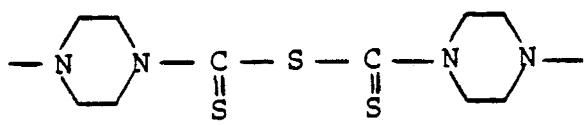

thiuram monosulfide

| (3) | $ZnC_{12}H_{22}N_4S_4$ | $ZnC_{12}H_{22}N_4S_4$ |
|---|---|---|

Structure and General Name

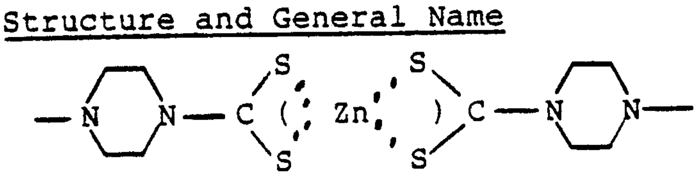

zinc dithiocarbamate

| (4) | $C_{12}H_{24}N_4S_2$ | $C_{12}H_{24}N_4S_2$ |
|---|---|---|

Structure and General Name

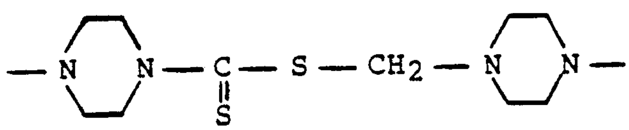

aminomethyl dithioester

| (5) | $C_6H_{12}N_2S_2$ | $C_6H_{12}N_2S_2$ |
|---|---|---|

Structure and General Name

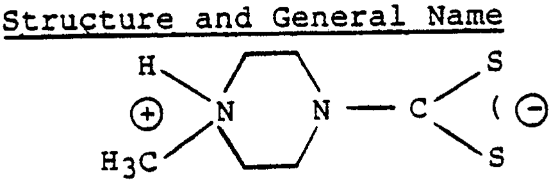

zwitterionic dithiocarbamate

Compounds 1,2, and 3 above are described (along with certain other metal dithiocarboxylates) by U.S. Patent No. 4,687,756. British Patent No. 1,426,857 describes compound 4. Compound 5 is a preferred embodiment of the present invention. For each of the Compounds 1,2,3, and 5, there is one mole of $CS_2$ incorporated into the molecule for each mole of 1-methylpiperazine, whereas for Compound 4, which is less active than the other four compounds, there is only 1/2 mole of $CS_2$ incorporated into the molecule per amine molecule.

## ADVANTAGES OF THE PRESENT INVENTION OVER THE PRIOR ART

The primary advantage of zwitterionic dithiocarbamates such as Compound 5 over conventional ditheriocarbamate accelerators such as Compounds 1,2, and 3 are decreased manufacturing costs and decreased chemical by-products of manufacture thereof. In terms of sulfur cure system activity, Compounds 1,2,3 and 5 all produce similar cure rate, but differ substantially in respect to scorch delay of rubber compounds incorporating these compounds. These compounds have increasing scorch safety in the order Compound 3<5<1<2. In those instances where the scorch safety afforded by Compound 5 is adequate, it would be favored on the basis of manufacturing cost, since each of the other compounds of similar activity (Compounds 1-3) requires additional reagents for synthesis thereof and each produces by-products (brine for all three; sodium thiocyanate typically for Compound 2).

## SUMMARY OF THE INVENTION

The polyamines utilized in producing the zwitterionic tertiary ammonium dithiocarbamates of the present invention contain at least one tertiary amine group covalently attached to a secondary amine. The compounds of the present invention are especially suitable as ultra-accelerators or as secondary accelerators in combination with 2-mercaptobenzothiazole derivatives in vulcanization of rubber, and yield unexpected improvements in the vulcanization process for rubber, especially with regard to cure rates such as indicated by $t_{90}$ minus $t_2$ values and especially $t_{25}$ minus $t_2$ values. Increased cure rates are desirable since faster rates of production of rubber articles can be obtained. The zwitterionic tertiary ammonium dithiocarbamates are also unusually simple to manufacture, and can be obtained in nearly quantitative yield by simple adduct formation between a suitable polyamine and $CS_2$.

## DETAILED DESCRIPTION

Zwitterionic tertiary ammonium dithiocarbamates are the reaction products of an amine compound with $CS_2$. The amine compound is a polyamine (that is having two or more amine groups) generally having at least one tertiary amine group and one secondary amine group therein. Such amine reactants can generally be represented by one of the four following structures:

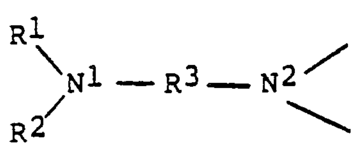

or

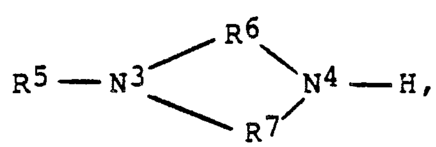

, or

Structure 1

Structure 2

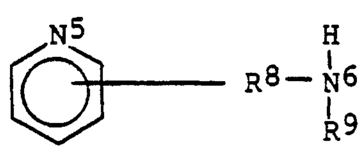

, or

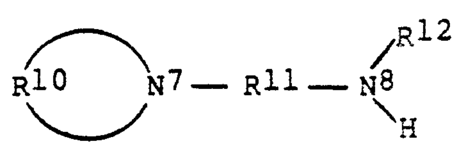

Structure 3

Structure 4

In the above structures, all the various R groups ($R^1$ to $R^{12}$) are connected to the various numbered nitrogen atoms through an $SP^3$-hybridized (aliphatic) carbon atom, which carbon atom is further not bonded to any highly electron-withdrawing chemical groups (such as $CF_3$). This requirement guarantees adequate basicity for the various amine groups.

$R^1$, $R^2$, $R^4$, $R^5$, $R^9$, and $R^{12}$, independently, can generally be a hydrocarbyl chemical group having from 1 to 100 carbon atoms with the proviso that there are no double bonds attached to the same carbon atoms of $R^1$, $R^2$, $R^4$, $R^5$, $R^9$ or $R^{12}$ which is covalently bonded to one of the numbered nitrogen atoms, $N^1$, $N^2$, $N^6$, or $N^8$. This also implies that no aryl group exists which is directly connected to a nitrogen atom. $R^1$, $R^2$, $R^4$, $R^5$, $R^9$, and $R^{12}$, independently, is desirably an alkyl group having from 1 to 18 carbon atoms, a cycloalkyl having from 5 to 10 carbon atoms, an aryl substituted alkyl having from 7 to 16 carbon atoms, or an

alkylene having from 2 to 10 carbon atoms, in which case the molecule is dimeric, containing two zwitterionic dithiocarbamate groups. The above-noted $R^1$, $R^2$, $R^4$, $R^5$, $R^9$, and $R^{12}$, groups, independently, can also be a hydrocarbyl containing functional groups therein and hence be an ester, an ether, an amide, a heteroaromatic, an aryl amine, a tertiary amine, a urea, a nitrile, a thiol, a thiourea, a disulfide, a carboxylic acid, and may contain other chemical groups which are not reactive with tertiary ammonium dithiocarbamates.

Among the various types of functional groups which may be contained in $R^1$, $R^2$, $R^4$, $R^5$, $R^9$, and $R^{12}$, are those types of chemical groups which serve a purpose in the rubber and which are preferred. For example, pyridyl, amide, or urea groups are known cure activators which may be incorporated into $R^1$, $R^2$, $R^4$, $R^5$, $R^9$, and $R^{12}$ to enhance cure activity of the chemicals of this invention in some cases. Antidegradant groups such as hindered phenolic, diaryl amine, or aryl-alkyl amine may also usefully be contained in $R^1$, $R^2$, $R^4$, $R^5$, $R^9$, and/or $R^{12}$, in which case the volatility of both the antidegradant and the cure accelerator are greatly reduced compared to the normal situation in which the antidegradant and the accelerator are separate molecules. Other purposes which may be served by functional groups contained in $R^1$, $R^2$, $R^4$, $R^5$, $R^9$, and/or $R^{12}$ are to decrease volatility (for which carboxylate salts are very effective), or to provide a means for grafting the molecule to rubber, for which a di- or poly-sulfide linkage is desirable. Representative examples of such $R^1$, $R^2$, $R^4$, $R^5$, $R^9$, and $R^{12}$ groups include methyl, ethyl, propyl, hexyl, octyl, benzyl, 2-hydroxyethyl, 2-furfuryl, 2-pyridylethyl, various 2-amidoethyl, 2-urea-substituted-ethyl, and 2-thiourea-substituted ethyl compounds,

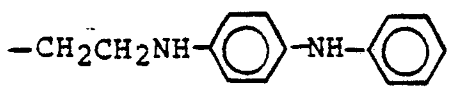 and other amine antioxidant groups;

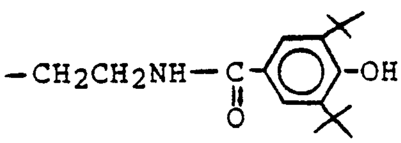 and other phenolic antioxidant groups;

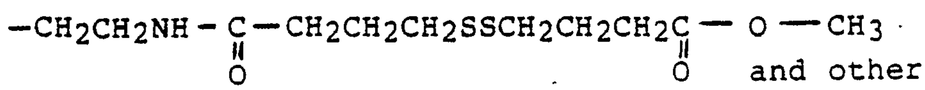 and other disulfides.

$R^1$, $R^2$, $R^4$, $R^5$, $R^9$, and $R^{12}$, independently, have a molecular weight up to about 350, except when $R^1$, $R^2$, $R^4$, $R^5$, $R^9$, and $R^{12}$ is an oligomer which may contain several functional groups such as those of Table I, and may also include about one to ten other dithiocarbamate groups, in which case the molecular weight of the oligomeric R group may be up to about 350 times the number of dithiocarbamate groups attached thereto.

## TABLE I

Representative organic chemical groups which may be $R^1$, $R^2$, $R^4$, $R^5$, $R^9$, and/or $R^{12}$.

(1) $-(CH_2)_n-R$ (n = 1 to 6; R is an aromatic group)
(2) $-(CH_2)_n-O-R$ (n = 1 to 6; R is an alkyl group or H)
(3)

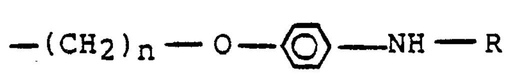

(n = 1 to 6; R is an alkyl or a cycloalkyl group)
(4) 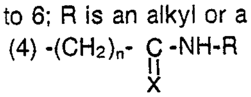

(n = 2 to 6; R can be an alkyl or aryl group, optionally containing functional groups; X can be oxygen or sulfur)

(5) $-(CH_2)_n-\underset{\underset{O}{\|}}{C}-O-R$

(n = 2 to 6; R is an alkyl group)

(6)

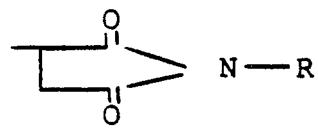

(R can be an alkyl or aryl group, optionally containing functional groups)

(7)

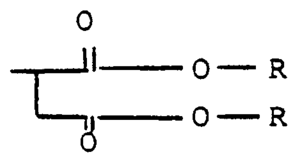

(R is an alkyl group)

(8) $-(CH_2)_n$ SH (n = 2 or 3)

(9) $-(CH_2)_n$ SSR (n = 2 or 3; R = various organic chemical groups)

(10)

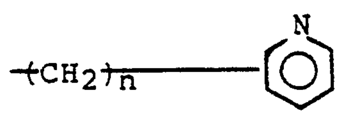

(n = 1 to 6)

(11) $-(CH)_nNH-\overset{\overset{O}{\|}}{C}-R$ (n = 2 to 6; R is alkyl, aryl, or aralkyl optionally containing other functional groups)

Preferred $R^1$, $R^2$, $R^4$, $R^5$, $R^9$ and $R^{12}$ groups, independently, include alkyl groups having from 1 to 6 carbon atoms; benzyl, esters derived from Michael addition reactions of polyamines to common monomers, such as butyl acrylate and other common acrylates; Formula 10 of Table I with n = 1 or 2; Formula 7 of Table I derived from Michael reactions of maleate diesters; Formula 5 and Formula 11 when R is an alkyl group having from 1 to 18 carbon atoms.

$R^3$, $R^8$, and $R^{11}$, independently, can generally be a hydrocarbyl or ether having from 1 to 12 carbon atoms and up to 5 ether oxygens, but desirably is an alkylene having from 1 to 6 carbon atoms or an alkyl ether having 2 to 6 carbon atoms and 1 or 2 oxygen atoms, and preferably is an alkylene having from 2 to 4 carbon atoms. Hence, ethylidene, n-propylidene, and n-butylidene are preferred groups.

$R^6$ and $R^7$, independently, is a hydrocarbyl having from 1 to 7 carbon atoms, desirably is an alkylene having from 1 to 3 carbon atoms, and preferably as an ethylidene group in which case Structure (2) corresponds to an N-substituted piperazine.

$R^{10}$ desirably is an alkylene having from 4 to 7 carbon atoms, or an ether of structure $-CH_2CH_2OCH_2CH_2-$.

Regardless of what types of $R^1$ through $R^{12}$ groups are utilized, it is an important aspect of the present invention that they not be a chemical moiety which is highly reactive with either tertiary ammonium cations or dithiocarbamate anions.

The zwitterionic tertiary ammonium dithiocarbamates of the present invention are preferably prepared by a direct reaction process. The reaction can take place either in a solvent or in a gas phase.

When a solvent route is used, suitable solvents include various hydrocarbon solvents such as the alkanes having from 5 to 9 carbon atoms as for example hexane, heptane, and the like, various hydrocarbon aromatics having from 6 to 12 carbon atoms such as toluene, benzene, xylenes, and the like; various petroleum distillates having flash points from -20° C to about 100° C; various chlorinated solvents which are not reactive with amines, such as $CH_2Cl_2$ and $CH_3CCl_3$; various alcohols, such as methanol, ethanol, and isopropanol; various ethers, such as diethyl ether, various glycol ethers; ketones such as acetone, methyl ethyl ketone, and methyl isobutyl ketone; excess $CS_2$; and various polymers (in which case adduct formation may occur in an extruder).

The amine compounds are preferably dissolved in the solvent and $CS_2$ subsequently added thereto, desirably with mixing. The reaction generally proceeds in a rapid manner and is usually carried out at a temperature of from about -20° C to about 120° C and preferably from about 10° to about 60° C. The reaction product is usually solid and can be recovered by a variety of methods as by filtration, centrifugation, and the like.

When the zwitterionic tertiary ammonium dithiocarbamate is made in a gas phase, the process is generally as follows: amine reactant vapor and $CS_2$ vapor are separately dissolved in a neutral diluent gas such as $N_2$, and the two gas solutions are mixed to form a smoke of very fine particle-size zwitterionic dithiocarbamate particles.

The zwitterionic tertiary ammonium dithiocarbamates of the present invention can be utilized as a primary accelerator or as a secondary accelerator in synthetic rubber and/or natural rubber. These zwitterionic dithiocarbamates are also useful as synthetic intermediates in the synthesis of a variety of dithiocarbamate derivatives. When utilized as a primary accelerator, the amount is generally from about 0.2 to about 5.0 and desirably from about 0.3 to about 3.0 parts by weight for every 100 parts by weight of rubber compound, depending on the nature of the elastomer (NR versus EPDM, for example), sulfur level, and the activity of the particular zwitterionic dithiocarbamate, as will be obvious to one skilled in the art of sulfur vulcanization of unsaturated elastomers. When utilized as a secondary or as a co-accelerator, the amount is generally from about 0.05 to about 2.0 and desirably from about 0.2 to about 1.0 parts by weight for every 100 parts by weight of the rubber elastomer.

Examples of sulfur-vulcanizable synthetic rubbers in which the adducts of the present invention can be utilized as accelerators include the so-called "diene-based elastomers", such as natural rubber (NR); diene homopolymers, such as those made from 1,3-butadiene (RR), isoprene (IR), and chloroprene (CR); and copolymers made from diene monomers such as copolymers of 1,3-butadiene and one or more non-diene monomers, such as styrene, alpha-methyl styrene, vinyl pyridines, acrylates, methacrylates, acrylonitrile, and the like. The copolymers preferably contain minor amounts of styrene acrylonitrile, or acrylates, and major amounts of butadiene. Such preferred copolymers include the well-known SBR and NBR copolymers. Both solution-polymerized and emulsion-polymerized polymers are included. Butadiene homopolymers include those having predominantly cis-1,4 microstructure, and homopolymers having substantial contents of trans- 1,4 and/or 1,2 enchained monomer units as well as cis-1,4 enchained butadiene units. The butadiene polymers used in the invention are preferably "elastomeric" materials; that is they conform, when vulcanized, to the definition of an elastomeric or rubbery material found in ASTM D 1566. As is well known to one skilled in the art of sulfur vulcanization, the various diene polymers discussed above vary substantially in activity towards sulfur vulcanization. The amount of zwitterionic dithiocarbamate accelerator required to effectively vulcanize these various elastomers increases in the order: NR, IR<BR<SBR<NBR.

Another class of synthetic rubbers which can be used in the present invention are various copolymer rubbers in which the content of vulcanizable chemical groups is much lower than for the aforesaid diene-based elastomers. Important examples of such rubbers include the isoprene/isobutene copolymers (IIR, or butyl rubber), and the EPDM rubbers. EPDM rubbers are polymers made from ethylene, propylene, and a non-conjugated diene monomer. Higher concentrations of accelerators are required to cure EPDM and IIR copolymer rubbers than those required for the diene-based elastomers. When such low-unsaturation elastomers as EPDM and IIR are sulfur vulcanized, they are nearly always accelerated with a synergistic combination of two or more accelerators. For these elastomers, the zwitterionic dithiocarbamates of the present invention are nearly always used in combination with one or more heteroaromatic thiol cure accelerators such as 2-mercaptobenzothiazole and its derivatives, various triazine thiol derivatives, and certain other nitrogen-containing heteroaromatic thiols and derivatives thereof, as will be familiar to one skilled in the art of sulfur vulcanization of EPDM and/or IIR.

Included within the definition of rubber compounds are blends of the aforesaid types of elastomers. Particularly significant examples of blends include NR/BR/SBR combinations; diene-based elastomers with EPDM; and IIR blends with EPDM and NBR.

The rubber polymers of the present invention are crosslinked by sulfur and/or by sulfur donors such as

dimorpholinodisulfide, various alkyl phenol disulfides, and/or various polysulfide-containing copolymers. Typically, low sulfur levels produce vulcanizates having low crosslink densities and a relatively low "state of cure," but with good resistance to aging. High amounts of sulfur, conversely, produce vulcanizates having high crosslink densities and a resultant high "state of cure," but with poor resistance to aging. Extremely high sulfur levels result in "hard rubber" or ebonite when used with diene-based elastomers. Preferred sulfur levels in the diene-based polymers are from 0.2 to 4 parts by weight per 100 parts by weight of polymer, depending on accelerator level and also on whether the sulfur is included as elemental sulfur or as a sulfur donor, which can be significantly more efficient as a sulfur source than elemental sulfur. When EPDM or IIR polymers are utilized, the amount of sulfur used is similar to the range cited above for the diene-based polymers, but total accelerator content is generally higher.

When the accelerators of this invention are utilized as secondary accelerators, the primary accelerator can be any conventional accelerator known to the art and to the literature. Such typical accelerators are listed in the "Rubber World Bluebook." Such accelerators include various heteroaromatic thiols and derivatives thereof, especially the thiazoles, such as 2-mercaptobenzothiazole and 2-benzothiazyl disulfide; benzothiazyl sulfenamides, such as N-cyclohexylbenzothiazylsulfenamide, N,N-dicyclohexylbenzothiazylsulfenamide, N,N-diethylbenzothiazylsulfenamide, N,N-diisopropylbenzothiazylsulfenamide, N-oxydiethylenebenzothiazylsulfenamide, N-isopropylbenzothiazylsulfenamide and N-t-butylbenzothiazylsulfenamide. When EPDM rubber is sulfur vulcanized, a thiazole accelerator is generally used in combination with a thiuram accelerator. The zwitterionic dithiocarbamates of this invention may be substituted for the thiuram accelerator, or may be used in addition to the thiazole thiuram combination system to achieve a faster cure rate. Examples of thiuram accelerators include tetramethylthiuram mono- and disulfides, dipentamethylenethiuram hexasulfide, tetrabutylthiuram mono- and disulfides, and tetraethylthiuram mono- and disulfides. Other conventional dithiocarbamates, such as metal salts of the corresponding dithiocarbamic acids, such as those of zinc, copper, tellurium, selenium, lead, and antimony, are also commonly used in vulcanization of EPDM and IIR.

In addition to the above compounds, the rubber compositions of the present invention can contain other conventional compounding ingredients well known to the art and to the literature. For example, various fillers and reinforcing agents, such as clay, silica, and carbon black, can be utilized in amounts up to about 200 phr. Various oils, for example aromatic, naphthenic, or paraffinic, can be utilized to plasticize the rubber in amounts up to about 200 phr. Various activators such as zinc oxide, stearic acid, and the like, can also be used in amounts up to about 15 or more phr. Various antidegradants, and the like, can also be utilized. Such materials are generally mixed into the rubber by utilizing a mill, a Banbury mixer, or the like.

The rubber compositions of the present invention can be used in a large number of applications including tires and various industrial rubber products. The zwitterionic dithiocarbamates of this invention are useful in tires primarily as secondary accelerators in relatively slow-curing (and also slow-scorching) SBR and/or SBR/BR stocks, as are often used in the tread and shoulder portions of tires. In general, the zwitterionic dithiocarbamates of the present invention are useful anywhere that dithiocarbamate accelerators are used at present.

Various zwitterionic tertiary ammonium dithiocarbamates of the present invention were tested in accordance with appropriate ASTM procedures for rubber. Parameters which characterize vulcanization were taken from ODR (oscillating disc rheometer) cure curves ("rheographs"), which were obtained for vulcanization at 153°C and/or 160°C. The parameters Rmin and Rmax are the minimum rheometer torque (before the onset of vulcanization) and the maximum rheometer torque (due to vulcanization), respectively. The parameter t2 is the time required for an increase (over Rmin) in rheometer torque of 0.223 joule (2.0 in-lb); t25 is the time required for the occurrence of 25 percent of the increase in torque due to vulcanization (time at which torque equals (Rmax-Rmin) 0.25 + Rmin); t90 is the time required for the occurrence of 90 percent of the increase in torque due to vulcanization (time at which torque equals (Rmax-Rmin) 0.9 + Rmin). Vmax is the maximum slope of the vulcani zation curve, expressed in terms of a percent of Rmax-Rmin per minute.

The invention will be better understood by reference to the following examples in which all parts are per 100 parts by weight of rubber (phr) and all temperatures are in degrees celsius, unless otherwise specified.

## Preparation of Rubber Masterbatches for Accelerator Evaluation

The various examples of zwitterionic tertiary ammonium dithiocarbamate accelerators which were prepared were tested in typical NR and SBR carbon-black reinforced compounds. Several of the most

active accelerators of this invention were subsequently tested in a typical EPDM composition.

An SBR rubber masterbatch was prepared, based on SBR-1500, and the SBR masterbatch contained the following ingredients:

| SBR Masterbatch | Parts |
|---|---|
| SBR-1500 | 100.0 |
| Carbon Black N-330 | 50.0 |
| Naphthenic Oil, ASTM D2226, Type 103 | 5.0 |
| Zinc Oxide | 5.0 |
| Stearic Acid | 2.0 |
| Total | 162.0 |

The SBR masterbatch was prepared by mixing the above-noted components in a Banbury mixer according to standard techniques. Subsequently, various accelerators, sulfur, and an antioxidant were added on a laboratory roll mill in the amounts set forth hereinbelow and blended by using standard laboratory mill mixing techniques.

| | Parts |
|---|---|
| SBR-Masterbatch | 166.0 |
| SANTOFLEX 13 | 2.0 |
| Sulfur | 2.0 |
| Accelerators | as indicated |

SBR-1500 is a cold emulsion-polymerized, non-pigmented styrene/butadiene copolymer rubber containing nominally 23.5 percent bound styrene;

SANTOFLEX® 13 is N-(1,3-dimethylbutyl)N′-phenyl-paraphenylenediamine, an antioxidant.

In a similar manner, a natural rubber masterbatch was made:

| Natural Rubber Masterbatch | Parts |
|---|---|
| Natural Rubber | 100.0 |
| Carbon Black N-330 | 50.0 |
| Naphthenic Oil; Circosol 4240 | 5.0 |
| Zinc Oxide | 5.0 |
| Stearic Acid | 2.0 |
| Total | 162.0 |

The natural rubber masterbatch was blended with the following compounds according to standard laboratory mill-mixing techniques.

| | Parts |
|---|---|
| Natural Rubber Masterbatch | 162.0 |
| SANTOFLEX 13 | 2.0 |
| Sulfur | 2.5 |
| Accelerators | as indicated |

Test data for various tertiary ammonium dithiocarbamates as SBR and NR accelerators are summarized in Table I. The control stock contained no zwitterionic tertiary ammonium dithiocarbamate. The control stock was prepared from the same masterbatch as was the experimental stock and measured in the same set on

the same day as was the experimental stock. The data of Table I shows various tertiary ammonium dithiocarbamates used as the only accelerator, and also shows that these accelerators are useful as secondary accelerators in combination with 2-mercaptobenzothiazole sulfenamides.

In a similar manner, an EPDM masterbatch was prepared. As is conventional for most EPDM compositions, this masterbatch contains a greater content of oil than of polymer:

| EPDM Rubber Masterbatch | |
|---|---|
| | Parts |
| Vistalon 5600 (EPDM) | 100 |
| Carbon Black N 550 | 100 |
| Carbon Black N 774 | 100 |
| Super 2280 (Paraffinic Oil) | 110 |
| Flectol H (antioxidant) | 2.0 |
| Zinc Oxide | 5.0 |
| Stearic Acid | 2.0 |

Unlike the previously described SBR and NR masterbatches, the EPDM masterbatch was mixed "upside down" in a laboratory Banbury mixer; that is, the carbon black and oil were added to the Banbury first, then the EPDM was added (as is conventional for highly extended EPDM compositions).

Subsequently, the EPDM masterbatch was blended with sulfur and accelerators on a laboratory mill according to standard laboratory millmixing techniques. Unlike the above experiments with NR and SBR, both sulfur and accelerator levels were varied, and complex synergistic mixtures of several accelerators were studied, as is conventional for EPDM vulcanizable compositions.

Various zwitterionic tertiary ammonium dithiocarbamates were prepared according to the present invention in the following manner.

## Illustrative Example 1

N-methylpiperazine was dissolved in hexane to yield approximately a 10 percent solution. This was added slowly with stirring to a stoichiometric equivalent of a 10 percent solution of carbon disulfide in hexane. The resultant adduct, 1-methylpiperazinium-4-dithiocarbamate, was obtained in 98 percent yield, and was found to be an excellent accelerator of sulfur vulcanization, and to be particularly effective in combination with accelerators based on 2-mercaptobenzothiazole.

## Illustrative Example 2

N-benzylpiperazine was dissolved in hexane and reacted with $CS_2$ as in Example 1. As was the case for the adduct of Example 1, N-benzylpiperazinium dithiocarbamate was also found to be an excellent accelerator of sulfur vulcanization, and to be particularly active in combination with sulfenamides.

## Illustrative Example 3

Butylacrylate was treated with excess piperazine to yield the Michael adduct, N-(butoxycarbonylethyl)-piperazine. This adduct reacted with $CS_2$ in toluene to yield a white precipitate which is very active as a sulfur vulcanization accelerator, and which is particularly active in combination with sulfenamide accelerators.

## Illustrative Example 4

1-(2-aminoethyl)piperazine was treated with a stoichiometric excess of 2,4-pentanedione. This reagent is known to add readily to primary amines but not to secondary amines. The reaction mixture was heated to drive off water and excess 2,4-pentanedione. Subsequently the corresponding imine:

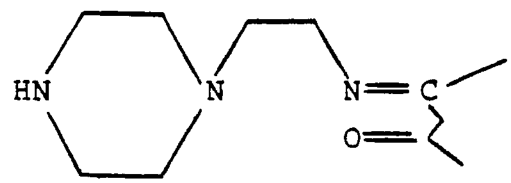

was treated with $CS_2$ in hexane to yield a yellow precipitate of the corresponding zwitterionic tertiary ammonium dithiocarbamate. This compound was an excellent accelerator of sulfur vulcanization, though it was somewhat less active than the compounds of Examples 1-3 as a sulfenamide synergist.

## Illustrative Example 5

A halogenated wax (Shattuck Chemical's Plastichlor 40-60® , 40 percent chlorine) was mixed with a tenfold excess of piperazine, and was subsequently washed with aqueous sodium hydroxide to remove unreacted piperazine and chloride ion. Substantially all of the organic halogen was removed from the wax either by substitution or elimination. This N-alkylpiperazine functional oligomer was treated with excess $CS_2$ in acetone solution, and then dried in a vacuum oven. The resultant oligomer was glassy at or slightly below room temperature, and showed moderate activity as an accelerator of sulfur vulcanization.

The above adducts of the present invention as well as other adducts similarly prepared were tested with regard to various accelerator properties and the results are set forth in Table II.

TABLE II

ODR DATA AT 153°C

| Type of Rubber | Control + SANTOCURE MOR (AMT) | 1-Methylpiperazinium-4-dithiocarbamate (AMT) |
|---|---|---|
| SBR | | |
| | (1.2 PHR) | (1.2 PHR) |
| Rmax, Nm | 3.86 | 4.15 |
| Rmin, Nm | 0.61 | 0.65 |
| t90, min. | 25.3 | 7.0 |
| t2, min. | 12.5 | 2.2 |
| t90-t2, min. | 12.8 | 4.8 |
| t25. min. | 15.5 | 2.7 |
| t25-t2, min. | 3.0 | 0.5 |
| Max Velocity, %/Min. | 12.5 | 52.8 |
| NATURAL RUBBER | (0.6 PHR) | (0.6 PHR) |
| Rmax, Nm | 3.64 | 3.72 |
| Rmin, Nm | 0.75 | 0.91 |
| t90, min. | 12.2 | 4.5 |
| t2, min. | 4.7 | 1.3 |
| t90-t2, min. | 7.5 | 3.2 |
| t25. min. | 6.2 | 1.8 |
| t25-t2, min. | 1.5 | 0.5 |
| Max Velocity, %/Min. | 18.2 | 52.5 |

TABLE II (continued)

ODR DATA AT 153°C

| Type of Rubber | Control + SANTOCURE MOR (AMT) | Piperazinium Dithio-carbamate (AMT) |
|---|---|---|
| SBR | (1.2 PHR) | (1.2 PHR) |
| Rmax, Nm | 3.86 | 2.94 |
| Rmin, Nm | 0.61 | 0.67 |
| t90, min. | 25.3 | 53.0 |
| t2, min. | 12.5 | 11.8 |
| t90-t2, min. | 12.8 | 41.2 |
| t25. min. | 15.5 | 18.0 |
| t25-t2, min. | 3.0 | 6.2 |
| Max Velocity, %/Min. | 12.5 | 2.8 |
| NATURAL RUBBER | (0.6 PHR) | (0.6 PHR) |
| Rmax, Nm | 3.64 | 2.57 |
| Rmin, Nm | 0.75 | 0.78 |
| t90, min. | 12.2 | 29.2 |
| t2, min. | 4.7 | 5.5 |
| t90-t2, min. | 7.5 | 23.7 |
| t25. min. | 6.2 | 7.8 |
| t25-t2, min. | 1.5 | 2.3 |
| Max Velocity, %/Min. | 18.2 | 5.1 |

TABLE II (continued)

ODR DATA AT 153°C

| Type of Rubber | Control + SANTOCURE MOR (AMT) | Benzylpiperaziniumdithiocarbamate (AMT) |
|---|---|---|
| SBR | (1.2 PHR) | (1.2 PHR) |
| Rmax, Nm | 4.13 | 3.80 |
| Rmin, Nm | 0.68 | 0.68 |
| t90, min. | 31.2 | 10.8 |
| t2, min. | 14.7 | 2.8 |
| t90-t2, min. | 16.5 | 8.0 |
| t25. min. | 19.0 | 3.5 |
| t25-t2, min. | 4.3 | 0.7 |
| Max Velocity, %/Min. | 9.9 | 33.6 |
| NATURAL RUBBER | (0.6 PHR) | (0.6 PHR) |
| Rmax, Nm | 3.59 | 3.27 |
| Rmin, Nm | 0.72 | 0.83 |
| t90, min. | 13.8 | 7.2 |
| t2, min. | 5.8 | 1.7 |
| t90-t2, min. | 8.0 | 5.5 |
| t25. min. | 7.0 | 2.2 |
| t25-t2, min. | 1.2 | 0.5 |
| Max Velocity, %/Min. | 16.0 | 34.5 |

TABLE II (continued)

ODR DATA AT 153°C

| Type of Rubber | Control + SANTOCURE MOR (AMT) | N-Carbobutoxy-ethyliminodi-thiocarbamate (AMT) |
|---|---|---|
| SBR | (1.2 PHR) | (1.2 PHR) |
| Rmax, Nm | 4.07 | 3.70 |
| Rmin, Nm | 0.67 | 0.70 |
| t90, min. | 28.7 | 13.3 |
| t2, min. | 13.5 | 3.5 |
| t90-t2, min. | 15.2 | 9.8 |
| t25. min. | 17.5 | 4.3 |
| t25-t2, min. | 4.0 | 0.8 |
| Max Velocity, %/Min. | 11.4 | 22.1 |
| NATURAL RUBBER | (0.6 PHR) | (0.6 PHR) |
| Rmax, Nm | 3.45 | 2.76 |
| Rmin, Nm | 0.67 | 0.77 |
| t90, min. | 13.5 | 10.8 |
| t2, min. | 5.2 | 2.5 |
| t90-t2, min. | 8.3 | 8.3 |
| t25. min. | 7.0 | 3.2 |
| t25-t2, min. | 1.8 | 0.7 |
| Max Velocity, %/Min. | 16.3 | 22.5 |

TABLE II (continued)

ODR DATA AT 153°C

| Type of Rubber | Control + SANTOCURE NS (AMT) | SANTOCURE NS and N-Methylpiperazinium-dithiocarbamate (AMT/AMT) |
|---|---|---|
| SBR | (1.2 PHR) | (1.2/0.5 PHR) |
| Rmax, Nm | 4.18 | 4.48 |
| Rmin, Nm | 0.65 | 0.64 |
| t90, min. | 26.7 | 11.5 |
| t2, min. | 12.0 | 6.2 |
| t90-t2, min. | 14.7 | 5.3 |
| t25. min. | 15.8 | 7.5 |
| t25-t2, min. | 3.8 | 1.3 |
| Max Velocity, %/Min. | 11.2 | 34.4 |
| NATURAL RUBBER | (0.6 PHR) | (0.6/0.5 PHR) |
| Rmax, Nm | 3.74 | 4.26 |
| Rmin, Nm | 0.67 | 0.73 |
| t90, min. | 13.7 | 3.8 |
| t2, min. | 5.7 | 2.3 |
| t90-t2, min. | 8.0 | 1.5 |
| t25. min. | 7.0 | 2.7 |
| t25-t2, min. | 1.3 | 0.4 |
| Max Velocity, %/Min. | 21.0 | 84.5 |

TABLE II(continued)

ODR DATA AT 153°C

| Type of Rubber | Control + SANTOCURE NS (AMT) | SANTOCURE NS and AK 8508-disulfide (AMT/AMT) |
|---|---|---|
| SBR | (1.2 PHR) | (1.2/0.5 PHR) |
| Rmax, Nm | 4.18 | 4.53 |
| Rmin, Nm | 0.65 | 0.65 |
| t90, min. | 26.7 | 13.0 |
| t2, min. | 12.0 | 7.0 |
| t90-t2, min. | 14.7 | 6.0 |
| t25. min. | 15.8 | 8.7 |
| t25-t2, min. | 3.8 | 1.7 |
| Max Velocity, %/Min. | 11.2 | 29.6 |
| NATURAL RUBBER | (0.6 PHR) | (0.6/0.5 PHR) |
| Rmax, Nm | 3.74 | 4.58 |
| Rmin, Nm | 0.67 | 0.74 |
| t90, min. | 13.7 | 4.5 |
| t2, min. | 5.8 | 2.8 |
| t90-t2, min. | 8.0 | 1.7 |
| t25. min. | 7.0 | 3.3 |
| t25-t2, min. | 1.3 | 0.5 |
| Max Velocity, %/Min. | 21.0 | 79.1 |

TABLE II(continued)

ODR DATA AT 153°C

| Type of Rubber | CONTROL + SANTOCURE NS (AMT) | SANTOCURE NS + CA(AK 8508)2 (AMT/AMT) |
|---|---|---|
| SBR | (1.2 PHR) | (1.2/0.5 PHR) |
| Rmax, Nm | 4.18 | 4.51 |
| Rmin, Nm | 0.65 | 0.64 |
| t90, min. | 26.7 | 12.2 |
| t2, min. | 12.0 | 6.7 |
| t90-t2, min. | 14.7 | 5.5 |
| t25. min. | 15.8 | 8.0 |
| t25-t2, min. | 3.8 | 1.3 |
| Max Velocity, %/Min. | 11.2 | 32.2 |
| NATURAL RUBBER | (0.6 PHR) | (0.6/0.5 PHR) |
| Rmax, Nm | 3.74 | 4.42 |
| Rmin, Nm | 0.67 | 0.70 |
| t90, min. | 13.7 | 4.3 |
| t2, min. | 5.7 | 2.8 |
| t90-t2, min. | 8.0 | 1.5 |
| t25. min. | 7.0 | 3.2 |
| t25-t2, min. | 1.3 | 0.4 |
| Max Velocity, %/Min. | 21.0 | 80.4 |

TABLE II (continued)

ODR DATA AT 153°C

| Type of Rubber | CONTROL + SANTOCURE NS (AMT) | SANTOCURE NS + 50% Ca(AK 8508)2 (AMT/AMT) |
|---|---|---|
| SBR | (1.2 PHR) | (1.2/0.5 PHR) |
| Rmax, Nm | 4.07 | 4.23 |
| Rmin, Nm | 0.61 | 0.61 |
| t90, min. | 25.8 | 16.3 |
| t2, min. | 12.0 | 8.3 |
| t90-t2, min. | 13.8 | 8.0 |
| t25. min. | 15.8 | 10.3 |
| t25-t2, min. | 3.8 | 2.0 |
| Max Velocity, %/Min. | 11.8 | 21.1 |
| NATURAL RUBBER | (0.6 PHR) | (0.6/0.5 PHR) |
| Rmax, Nm | 3.56 | 3.99 |
| Rmin, Nm | 0.56 | 0.60 |
| t90, min. | 13.2 | 6.3 |
| t2, min. | 6.0 | 3.8 |
| t90-t2, min. | 7.2 | 2.5 |
| t25. min. | 7.3 | 4.2 |
| t25-t2, min. | 1.3 | 0.4 |
| Max Velocity, %/Min. | 22.4 | 71.8 |

TABLE II (continued)

ODR DATA AT 153°C

| Type of Rubber | CONTROL + SANTOCURE NS (AMT) | SANTOCURE NS + Piperazinium dithiocarbamate (AMT/AMT) |
|---|---|---|
| SBR | (1.2 PHR) | (1.2/0.5 PHR) |
| Rmax, Nm | 4.12 | 4.34 |
| Rmin, Nm | 0.67 | 0.68 |
| t90, min. | 24.2 | 16.2 |
| t2, min. | 11.0 | 7.8 |
| t90-t2, min. | 13.2 | 8.4 |
| t25. min. | 14.5 | 9.5 |
| t25-t2, min. | 3.5 | 1.7 |
| Max Velocity, %/Min. | 13.2 | 21.2 |
| NATURAL RUBBER | (0.6 PHR) | (0.6/0.5 PHR) |
| Rmax, Nm | 3.75 | 4.10 |
| Rmin, Nm | 0.61 | 0.65 |
| t90, min. | 13.0 | 9.5 |
| t2, min. | 5.8 | 4.5 |
| t90-t2, min. | 7.2 | 5.0 |
| t25. min. | 7.2 | 5.3 |
| t25-t2, min. | 1.4 | 0.8 |
| Max Velocity, %/Min. | 23.0 | 34.4 |

TABLE II (continued)

ODR DATA AT 153°C

| Type of Rubber | CONTROL + SANTOCURE NS (AMT) | SANTOCURE NS + N-carbobutoxy-ethylimino-dithiocarbamate (AMT/AMT) |
|---|---|---|
| SBR | (1.2 PHR) | (1.2/0.5 PHR) |
| Rmax, Nm | 4.10 | 4.27 |
| Rmin, Nm | 0.66 | 0.67 |
| t90, min. | 25.5 | 16.7 |
| t2, min. | 11.5 | 8.3 |
| t90-t2, min. | 14.0 | 8.4 |
| t25. min. | 15.2 | 10.2 |
| t25-t2, min. | 3.7 | 1.9 |
| Max Velocity, %/Min. | 12.1 | 21.3 |
| NATURAL RUBBER | (0.6 PHR) | (0.6/0.5 PHR) |
| Rmax, Nm | 3.79 | 4.16 |
| Rmin, Nm | 0.65 | 0.70 |
| t90, min. | 13.0 | 6.3 |
| t2, min. | 5.8 | 3.2 |
| t90-t2, min. | 7.2 | 3.1 |
| t25. min. | 7.2 | 3.8 |
| t25-t2, min. | 1.4 | 0.6 |
| Max Velocity, %/Min. | 22.5 | 51.1 |

TABLE II (continued)

ODR DATA AT 153°C

| Type of Rubber | CONTROL + SANTOCURE MOR (AMT) | SANTOCURE MOR + N-carbobutoxy-ethyliminodi-thiocarbamate (AMT/AMT) |
|---|---|---|
| SBR | (1.2 PHR) | (1.2/2.0 PHR) |
| Rmax, Nm | 4.10 | 4.98 |
| Rmin, Nm | 0.69 | 0.69 |
| t90, min. | 30.0 | 9.2 |
| t2, min. | 14.0 | 4.2 |
| t90-t2, min. | 16.0 | 5.0 |
| t25. min. | 18.0 | 4.8 |
| t25-t2, min. | 4.0 | 0.6 |
| Max Velocity, %/Min. | 10.3 | 51.6 |
| NATURAL RUBBER | (0.6 PHR) | (0.6/2.0 PHR) |
| Rmax, Nm | 3.61 | 4.58 |
| Rmin, Nm | 0.76 | 0.78 |
| t90, min. | 13.8 | 2.7 |
| t2, min. | 5.2 | 1.3 |
| t90-t2, min. | 8.6 | 1.4 |
| t25. min. | 7.0 | 1.7 |
| t25-t2, min. | 1.8 | 0.4 |
| Max Velocity, %/Min. | 16.1 | 85.7 |

TABLE II (continued)

ODR DATA AT 153°C

| Type of Rubber | CONTROL + SANTOCURE NS (AMT) | SANTOCURE NS + 1-Methylpiperaziniumdithiocarbamate (AMT/AMT) |
|---|---|---|
| SBR | (1.2 PHR) | (1.2/0.5 PHR) |
| Rmax, Nm | 4.10 | 4.54 |
| Rmin, Nm | 0.66 | 0.67 |
| t90, min. | 25.5 | 11.8 |
| t2, min. | 11.5 | 6.5 |
| t90-t2, min. | 14.0 | 5.3 |
| t25. min. | 15.2 | 7.7 |
| t25-t2, min. | 3.7 | 1.2 |
| Max Velocity, %/Min. | 12.1 | 34.9 |
| NATURAL RUBBER | (0.6 PHR) | (0.6/0.5 PHR) |
| Rmax, Nm | 3.79 | 4.39 |
| Rmin, Nm | 0.65 | 0.68 |
| t90, min. | 13.0 | 3.7 |
| t2, min. | 5.8 | 2.2 |
| t90-t2, min. | 7.2 | 1.5 |
| t25. min. | 7.2 | 2.5 |
| t25-t2, min. | 1.4 | 0.3 |
| Max Velocity, %/Min. | 22.5 | 83.7 |

TABLE II (continued)

ODR DATA AT 153°C

| Type of Rubber | CONTROL + SANTOCURE NS (AMT) | SANTOCURE NS + 1-Benzylpiperaziniumdithiocarbamate (AMT/AMT) |
|---|---|---|
| SBR | (1.2 PHR) | 1.2/0.5 PHR) |
| Rmax, Nm | 4.10 | 4.29 |
| Rmin, Nm | 0.66 | 0.66 |
| t90, min. | 25.5 | 16.0 |
| t2, min. | 11.5 | 8.3 |
| t90-t2, min. | 14.0 | 7.7 |
| t25. min. | 15.2 | 10.0 |
| t25-t2, min. | 3.7 | 1.7 |
| Max Velocity, %/Min. | 12.1 | 23.0 |
| NATURAL RUBBER | (0.6 PHR) | (0.6/0.5 PHR) |
| Rmax, Nm | 3.79 | 4.27 |
| Rmin, Nm | 0.65 | 0.65 |
| t90, min. | 13.0 | 5.3 |
| t2, min. | 5.8 | 2.8 |
| t90-t2, min. | 7.2 | 2.5 |
| t25. min. | 7.2 | 3.3 |
| t25-t2, min. | 1.4 | 0.5 |
| Max Velocity, %/Min. | 22.5 | 65.0 |

TABLE II (continued)

ODR DATA AT 153°C

| Type of Rubber | CONTROL + SANTOCURE NS (AMT) | SANTOCURE NS + Piperazinium dithiocarbamate (AMT/AMT) |
|---|---|---|
| SBR | (1.2 PHR) | 1.2/0.5 PHR) |
| Rmax, Nm | 4.12 | 4.34 |
| Rmin, Nm | 0.67 | 0.68 |
| t90, min. | 24.2 | 16.2 |
| t2, min. | 11.0 | 7.8 |
| t90-t2, min. | 13.2 | 8.4 |
| t25. min. | 14.5 | 9.5 |
| t25-t2, min. | 3.5 | 1.7 |
| Max Velocity, %/Min. | 13.2 | 21.2 |
| NATURAL RUBBER | (0.6 PHR) | (0.6/0.5 PHR) |
| Rmax, Nm | 3.75 | 4.10 |
| Rmin, Nm | 0.61 | 0.65 |
| t90, min. | 13.0 | 9.5 |
| t2, min. | 5.8 | 4.5 |
| t90-t2, min. | 7.2 | 5.0 |
| t25. min. | 7.2 | 5.3 |
| t25-t2, min. | 1.4 | 0.8 |
| Max Velocity, %/Min. | 23.0 | 34.4 |

TABLE II (continued)

ODR DATA AT 153°C

| Type of Rubber | CONTROL + SANTOCURE NS (AMT) | SANTOCURE NS + N-Methylpiperaziniumdithiocarbamate (AK 8508) (AMT/AMT) |
|---|---|---|
| SBR | (1.2 PHR) | 1.2/0.5 PHR) |
| Rmax, Nm | 4.18 | 4.48 |
| Rmin, Nm | 0.65 | 0.64 |
| t90, min. | 26.7 | 11.5 |
| t2, min. | 12.0 | 6.2 |
| t90-t2, min. | 14.7 | 5.3 |
| t25. min. | 15.8 | 7.5 |
| t25-t2, min. | 3.8 | 1.3 |
| Max Velocity, %/Min. | 11.2 | 34.4 |
| NATURAL RUBBER | (0.6 PHR) | (0.6/0.5 PHR) |
| Rmax, Nm | 3.74 | 4.26 |
| Rmin, Nm | 0.67 | 0.73 |
| t90, min. | 13.7 | 3.8 |
| t2, min. | 5.7 | 2.3 |
| t90-t2, min. | 8.0 | 1.5 |
| t25. min. | 7.0 | 2.7 |
| t25-t2, min. | 1.3 | 0.4 |
| Max Velocity, %/Min. | 21.0 | 84.5 |

TABLE II (continued)

ODR DATA AT 153°C

| Type of Rubber | CONTROL + SANTOCURE NS (AMT) | SANTOCURE NS + CA (AK 8508)2 (AMT/AMT) |
|---|---|---|
| SBR | (1.2 PHR) | 1.2/0.5 PHR) |
| Rmax, Nm | 4.18 | 4.51 |
| Rmin, Nm | 0.65 | 0.64 |
| t90, min. | 26.7 | 12.2 |
| t2, min. | 12.0 | 6.7 |
| t90-t2, min. | 14.7 | 5.5 |
| t25. min. | 15.8 | 8.0 |
| t25-t2, min. | 3.8 | 1.3 |
| Max Velocity, %/Min. | 11.2 | 32.2 |
| NATURAL RUBBER | (0.6 PHR) | (0.6/0.5 PHR) |
| Rmax, Nm | 3.74 | 4.42 |
| Rmin, Nm | 0.67 | 0.70 |
| t90, min. | 13.7 | 4.3 |
| t2, min. | 5.7 | 2.8 |
| t90-t2, min. | 8.0 | 1.5 |
| t25. min. | 7.0 | 3.2 |
| t25-t2, min. | 1.3 | 0.4 |
| Max Velocity, %/Min. | 21.0 | 80.4 |

TABLE II (continued)

ODR DATA AT 153°C

| Type of Rubber | CONTROL + SANTOCURE NS (AMT) | SANTOCURE NS + AK 8508 Disulfide (AMT/AMT) |
|---|---|---|
| SBR | (1.2 PHR) | 1.2/0.5 PHR) |
| Rmax, Nm | 4.18 | 4.53 |
| Rmin, Nm | 0.65 | 0.65 |
| t90, min. | 26.7 | 13.0 |
| t2, min. | 12.0 | 7.0 |
| t90-t2, min. | 14.7 | 6.0 |
| t25. min. | 15.8 | 8.7 |
| t25-t2, min. | 3.8 | 1.7 |
| Max Velocity, %/Min. | 11.2 | 29.6 |
| NATURAL RUBBER | (0.6 PHR) | (0.6/0.5 PHR) |
| Rmax, Nm | 3.74 | 4.58 |
| Rmin, Nm | 0.67 | 0.74 |
| t90, min. | 13.7 | 4.5 |
| t2, min. | 5.7 | 2.8 |
| t90-t2, min. | 8.0 | 1.7 |
| t25. min. | 7.0 | 3.3 |
| t25-t2, min. | 1.3 | 0.5 |
| Max Velocity, %/Min. | 21.0 | 79.1 |

TABLE II (continued)

ODR DATA AT 153°C

| Type of Rubber | CONTROL + SANTOCURE NS (AMT) | SANTOCURE NS + 50% CA (AK 8508)2 (AMT/AMT) |
|---|---|---|
| SBR | (1.2 PHR) | 1.2/1.0 PHR) |
| Rmax, Nm | 4.07 | 4.48 |
| Rmin, Nm | 0.61 | 0.62 |
| t90, min. | 25.8 | 11.8 |
| t2, min. | 12.0 | 6.5 |
| t90-t2, min. | 13.8 | 5.3 |
| t25. min. | 15.8 | 7.8 |
| t25-t2, min. | 3.8 | 1.3 |
| Max Velocity, %/Min. | 11.8 | 32.6 |
| NATURAL RUBBER | (0.6 PHR) | (0.6/1.0 PHR) |
| Rmax, Nm | 3.56 | 4.19 |
| Rmin, Nm | 0.56 | 0.59 |
| t90, min. | 13.2 | 4.3 |
| t2, min. | 6.0 | 2.8 |
| t90-t2, min. | 7.2 | 1.5 |
| t25. min. | 7.3 | 3.2 |
| t25-t2, min. | 1.3 | 0.4 |
| Max Velocity, %/Min. | 22.4 | 102.5 |

TABLE II (continued)

ODR DATA AT 153°C

| Type of Rubber | CONTROL + SANTOCURE (AMT) | SANTOCURE + 1-Methylpiperazinium dithiocarbamate (AMT/AMT) | |
|---|---|---|---|
| SBR | (1.2 PHR) | (1/2/0.1 PHR) | (1.2/0.2 PHR) |
| Rmax, Nm | 3.86 | 3.93 | 4.00 |
| Rmin, Nm | 0.67 | 0.64 | 0.65 |
| t90, min. | 21.7 | 19.2 | 15.5 |
| t2, min. | 9.4 | 8.7 | 7.3 |
| t90-t2, min. | 12.3 | 10.5 | 8.2 |
| t25 min. | 11.5 | 10.5 | 8.7 |
| t25-t2, min. | 2.1 | 1.8 | 1.4 |
| Max Velocity, %/Min. | 12.2 | 15.4 | 20.7 |
| NATURAL RUBBER | (0.6 PHR) | (0.6/0.2 PHR) | |
| Rmax, Nm | 3.67 | 3.96 | |
| Rmin, Nm | 0.70 | 0.73 | |
| t90, min. | 12.0 | 6.2 | |
| t2, min. | 5.2 | 3.0 | |
| t90-t2, min. | 6.8 | 3.2 | |
| t25. min. | 6.1 | 3.5 | |
| t25-t2, min. | 0.9 | 0.5 | |
| Max Velocity, %/Min. | 24.1 | 57.0 | |

TABLE II (continued)

ODR DATA AT 153°C

| Type of Rubber | CONTROL + SANTOCURE (AMT) | SANTOCURE + Piperazinium dithiocarbamate (AMT/AMT) |
|---|---|---|
| SBR | (1.2/0.2 PHR) | (1.2/0.2 PHR) |
| Rmax, Nm | 3.86 | 4.03 |
| Rmin, Nm | 0.67 | 0.66 |
| t90, min. | 21.7 | 16.8 |
| t2, min. | 9.4 | 7.7 |
| t90-t2, min. | 12.3 | 9.1 |
| t25. min. | 11.5 | 9.2 |
| t25-t2, min. | 2.1 | 1.5 |
| Max Velocity, %/Min. | 12.2 | 18.1 |
| NATURAL RUBBER | (0.6 PHR) | (0.6/0.2 PHR) |
| Rmax, Nm | 3.67 | 3.92 |
| Rmin, Nm | 0.70 | 0.72 |
| t90, min. | 12.0 | 9.2 |
| t2, min. | 5.2 | 4.0 |
| t90-t2, min. | 6.8 | 5.2 |
| t25. min. | 6.1 | 4.8 |
| t25-t2, min. | 0.9 | 0.8 |
| Max Velocity, %/Min. | 24.1 | 35.1 |

As is apparent from Table II, the use of the zwitterionic tertiary ammonium dithiocarbamate compounds of the present invention yielded improved cure rates, i.e., lower values of t90 minus t2 as well as t25 minus t2 when utilized with both styrene-butadiene rubber and natural rubber. Such increases were noted when utilized as a primary accelerator as well as a secondary accelerator.

While in accordance with the Patent Statutes, the best mode and preferred embodiment has been set forth, the scope of the invention is not limited thereto, but rather by the scope of the attached claims.

## Claims

1. A zwitterionic tertiary ammonium dithiocarbamate, comprising the reaction product of a polyamine and $CS_2$ having a tertiary ammonium cation and a dithiocarbamate anion therein, said polyamine having at least one tertiary amine and at least one secondary amine, and said tertiary ammonium cation being covalently bonded to said dithiocarbamate anion.

2. A zwitterionic tertiary ammonium dithiocarbamate according to Claim 1, wherein said amine compound has the formula

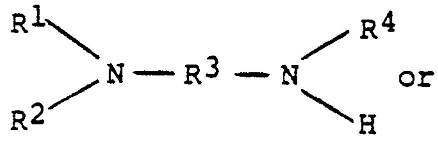 or 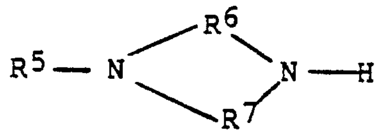, or

Structure 1 Structure 2

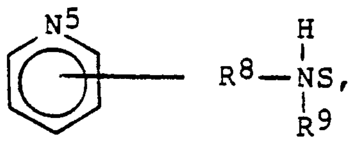 or 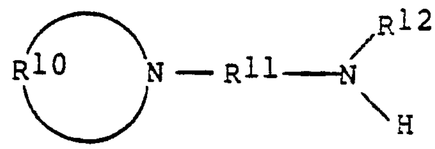

Structure 3 Structure 4

wherein $R^1$, $R^2$, and $R^4$, $R^5$, $R^9$, and $R^{12}$, independently, is a hydrocarbyl having from 1 to 100 carbon atoms with the proviso that no alpha double bond exists and that no aryl group exists which is directly connected to a nitrogen atom,

wherein $R^3$, $R^8$, and $R^{11}$, independently, is a hydrocarbyl or an ether having from 1 to 12 carbon atoms and up to 5 ether oxygen atoms,

wherein $R^6$ and $R^7$, independently, is a hydrocarbyl having from 1 to 7 carbon atoms, and

wherein $R^{10}$ is an alkylene having from 4 to 7 carbon atoms.

3. A zwitterionic tertiary ammonium dithiocarbamate according to Claim 2, wherein $R^1$, $R^2$, $R^4$, $R^5$, $R^9$, and $R^{12}$, independently, is an alkyl or aralkyl group having from 1 to 7 carbon atoms, a cycloalkyl having from 5 to 10 carbon atoms, an aryl having from 7 to 16 carbon atoms, an aryl substituted alkyl or an alkyl substituted aryl having from 7 to 16 carbon atoms, an alkylene having from 2 to 10 carbon atoms, and optionally a functional group selected from the class consisting of an ester, an ether, an amide, a heteroaromatic, an aryl amine, a tertiary amine, a urea, a nitrile, a thiol, a thiourea, a disulfide, or a carboxylic acid.

4. A zwitterionic tertiary ammonium dithiocarbamate according to Claim 3, wherein $R^3$, $R^8$, and $R^{11}$, independently, is an alkylene having from 1 to 6 carbon atoms or an alkyl ether having from 2 to 6 carbon atoms and 1 or 2 oxygen atoms, and wherein $R^6$ and $R^7$, independently, is an alkylene having from 1 to 6 carbon atoms.

5. A zwitterionic tertiary ammonium dithiocarbamate according to Claim 4, wherein $R^1$, $R^2$, $R^4$, $R^5$, $R^9$, and $R^{12}$, independently, is

(1)

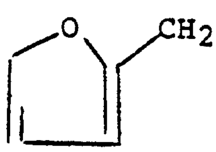 ;

(2) $-CH_2CH_2-O-R$

wherein R is an alkyl group or H;

(3)

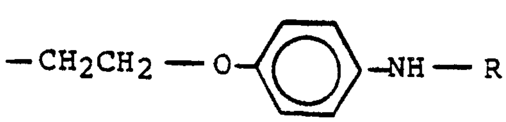

wherein R is an alkyl or a cycloalkyl group;

(4) $-CH_2CH_2NH-\underset{\underset{X}{\|}}{C}-NH-R$

wherein R can be an alkyl or aryl group, optionally containing functional groups, and X can be oxygen or sulfur;

(5) 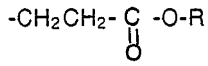

wherein R is an alkyl group;

(6)

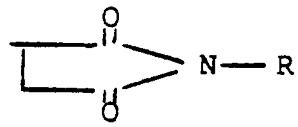

wherein R can be an alkyl or aryl group, optionally containing functional groups;

(7)

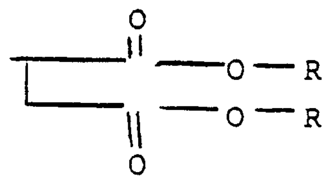

wherein R is an alkyl group;

(8) $\leftarrow(CH_2)_n$ SH wherein n = 2 or 3;

(9) $\leftarrow(CH_2)_n$ SSR

wherein n = 2 or 3; R = various organic chemical groups;

(10) 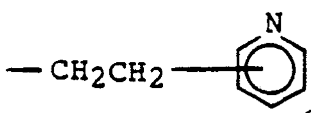 ; and

(11) 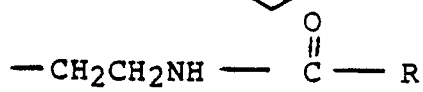

wherein R is alkyl, aryl, or aralkyl optionally containing other functional groups.

6. A zwitterionic tertiary ammonium dithiocarbamate according to Claim 5, wherein $R^3$, $R^8$, and $R^{11}$, independently, is an alkylene having from 2 to 4 carbon atoms, and wherein $R^6$ and $R^7$, independently, is ethylidene.

7. A vulcanizable composition, comprising a sulfur-vulcanizable synthetic rubber, sulfur, and an effective amount of an accelerator or a co-accelerator, said accelerator being a zwitterionic tertiary ammonium dithiocarbamate as set forth in Claim 1.

8. A vulcanizable composition, comprising a sulfur-vulcanizable synthetic rubber, sulfur, and an effective amount of an accelerator or a co-accelerator, said accelerator being a zwitterionic tertiary ammonium dithiocarbamate as set forth in Claim 2.

9. A vulcanizable composition, comprising a sulfur-vulcanizable synthetic rubber, sulfur, and an effective amount of an accelerator or a co-accelerator, said accelerator being a zwitterionic tertiary ammonium dithiocarbamate as set forth in Claim 3.

10. A vulcanizable composition, comprising a sulfur-vulcanizable synthetic rubber, sulfur, and an effective amount of an accelerator or a co-accelerator, said accelerator being a zwitterionic tertiary ammonium dithiocarbamate as set forth in Claim 4.

11. A vulcanizable composition, comprising a sulfur-vulcanizable synthetic rubber, sulfur, and an effective amount of an accelerator or a co-accelerator, said accelerator being a zwitterionic tertiary ammonium dithiocarbamate as set forth in Claim 5.

12. A vulcanizable composition, com prising a sulfur-vulcanizable synthetic rubber, sulfur, and an effective amount of an accelerator or a co-accelerator, said accelerator being a zwitterionic tertiary ammonium dithiocarbamate as set forth in Claim 6.

13. A process for preparing a zwitterionic tertiary ammonium dithiocarbamate compound, comprising the steps of:

mixing and reacting a polyamine with $CS_2$ and producing a product containing a tertiary ammonium cation and wherein said $CS_2$ becomes part of a N,N-dialiphatic dithiocarbamate anion which is covalently bonded

to said tertiary ammonium cation, said polyamine having the formula

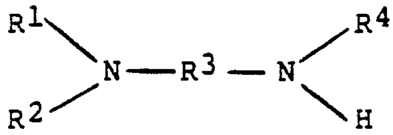 or 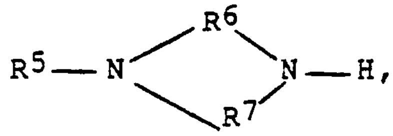, or

Structure 1 Structure 2

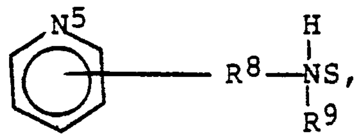, or 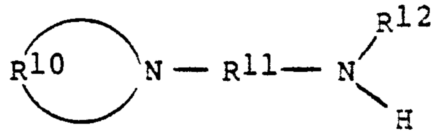

Structure 3 Structure 4

wherein $R^1$, $R^2$, and $R^4$, $R^5$, $R^9$, and $R^{12}$, independently, is a hydrocarbyl having from 1 to 100 carbon atoms with the proviso that no alpha double bond exists and that no aryl group exists which is directly connected to a nitrogen atom,
wherein $R^3$, $R^8$, and $R^{11}$, independently, is a hydrocarbyl or an ether having from 1 to 12 carbon atoms and up to 5 ether oxygen atoms,
$R^6$ and $R^7$, independently, is a hydrocarbyl having from 1 to 7 carbon atoms, and
wherein $R^{10}$ is an alkylene having from 4 to 7 carbon atoms.

14. A process according to Claim 13, wherein said reaction occurs in a non-hydroxylic solvent or in a gas.

15. A process according to Claim 14, wherein $R^1$, $R^2$, $R^4$, $R^5$, $R^9$, and $R^{12}$, independently, is an alkyl or aralkyl group having from 1 to 7 carbon atoms, a cycloalkyl having from 5 to 10 carbon atoms, an aryl having from 7 to 16 carbon atoms, an aryl substituted alkyl or an alkyl substituted aryl having from 7 to 16 carbon atoms, an alkylene having from 2 to 10 carbon atoms, and optionally a functional group selected from the class consisting of an ester, an ether, an amide, a heteroaromatic, an aryl amine, a tertiary amine, a urea, a nitrile, a thiol, a thiourea, a disulfide, or a carboxylic acid, and including carrying out said reaction at a temperature of from about minus 20 °C to about 120 °C.

16. A process according to Claim 15, wherein $R^3$, $R^8$, and $R^{11}$, independently, is an alkylene having from 1 to 6 carbon atoms or an alkyl ether having from 2 to 6 carbon atoms and 1 or 2 oxygen atoms, and wherein $R^6$ and $R^7$, independently, is an alkylene having from 1 to 6 carbon atoms.

17. A process according to Claim 16, wherein $R^1$, $R^2$, $R^4$, $R^5$, $R^9$, and $R^{12}$, independently, is

(1)

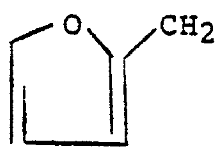 ;

(2) $-CH_2CH_2-O-R$
wherein R is an alkyl group or H;

(3)

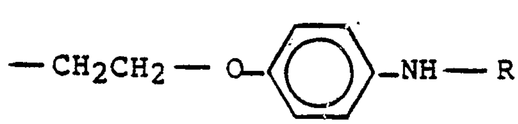

wherein R is an alkyl or a cycloalkyl group;

(4) $CH_2CH_2NH$- C -NH-R
||
X

wherein R can be an alkyl or aryl group, optionally containing functional groups, and X can be oxygen or sulfur;

(5) $CH_2CH_2-\underset{\underset{O}{||}}{C}-O-R$

wherein R is an alkyl group;

(6)

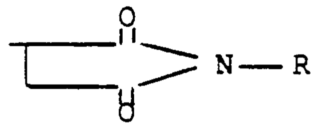

wherein R can be an alkyl or aryl group, optionally containing functional groups;

(7)

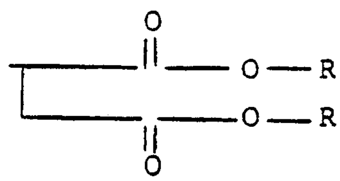

wherein R is an alkyl group;

(8) $-(CH_2)_n SH$ wherein n = 2 or 3;

(9) $-(CH_2)_n SSR$

wherein n = 2 or 3; R = various organic chemical groups;

(10) 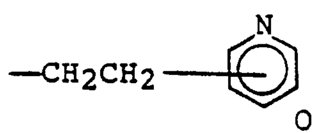 ; and

(11) $-CH_2CH_2NH-\overset{\overset{O}{||}}{C}-R$

wherein R is alkyl, aryl, or aralkyl optionally containing other functional groups.

18. A process according to Claim 17, wherein $R^3$, $R^8$, and $R^{11}$, independently, is an alkylene having from 2 to 4 carbon atoms, and wherein $R^6$ and $R^7$, independently, is ethylidene.

19. A process according to Claim 14, wherein said product is recovered by filtration.

20. A process according to Claim 17, wherein said reaction temperature is from about 10 °C to about 60 °C, and including recovering said product by filtration.

European Patent Office

# EUROPEAN SEARCH REPORT

Application Number

EP 90 87 0077

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-1 810 664 (AB ASTRA)<br>* Page 28, example 12; page 30, table I, lines 2,3; page 32, table II, lines 1,3,4,5,11,13,14; page 33, line 4 *<br>--- | 1-4,13-15 | C 07 D 295/20<br>C 07 C 333/16<br>C 08 L 21/00<br>C 08 K 5/39 |
| D,Y | US-A-4 687 756 (A. OKAMOTO et al.)<br>* Claims *<br>--- | 1-4,7-16,19 | |
| Y | US-A-2 905 655 (H.E. ALBERT)<br>* Columns 3,4, examples; claims *<br>--- | 1-4,7-16,19 | |
| A | JOURNAL OF ORGANIC CHEMISTRY, vol. 38, no. 3, 1973, pages 560-563, Washington, DC, US; D. DE FILIPPO et al.: "Inductive effect in dithiocarbamate decomposition mechanism"<br>--- | | |
| A | US-A-2 729 645 (H.L. KLOPPING)<br>--- | | |
| A | US-A-4 009 130 (R.L. ZIMMERMAN et al.)<br>----- | | TECHNICAL FIELDS SEARCHED (Int. Cl.5)<br>C 07 C 333/00<br>C 07 C 335/00<br>C 07 D 295/00<br>C 08 K 5/00 |

The present search report has been drawn up for all claims

| Place of search | Date of completion of the search | Examiner |
|---|---|---|
| THE HAGUE | 04-08-1990 | ZAROKOSTAS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document